# EUROPEAN PATENT APPLICATION

(11) **EP 4 843 516 A2**
(43) Date of publication of application: **25.11.2026**
(21) Application number: 26211453.1
(22) Date of filing: 03.05.2024
(51) Int. Cl.: A61B 17/11

(54) **CONNECTOR FOR AORTA CONNECTION**

(30) Priority: 11.05.2023 IT 202300009384
(62) Document number(s) of the earlier application(s) in accordance with Art. 76 EPC: 24730446.2 / 4 709 294
(71) Applicant: Phoenix S.r.l., 41038 San Felice Sul Panaro (IT)
(72) Inventor: Gazzani, Romolo, San Felice sul Panaro (MO) (IT); Bisio, Luigi, San Felice sul Panaro (MO) (IT)
(74) Representative: Garavelli, Paolo

(57) **Abstract**

A connector (10') for aorta connection is described, comprising: a sleeve (11) having a first mouth (12) to be connected to an aorta (21), and a second mouth (14) to be connected to a duct; a cylindrical lining element (50) placed around and outside of the sleeve (11); an annular locking flange (52) placed around and outside the cylindrical covering element (50); a retaining ring (51), placed around and outside the aorta (21) to fix the aorta (21) to the sleeve (11); and fixing means (41) of the retaining ring (51) to the aorta (21).

## Description

The present invention refers to a connector for aorta connection.

In general, the present invention refers to a connector for connecting two conduits for anastomosis operations, for example for connecting an aorta to a prosthetic tube.

More specifically, the invention concerns a connector connecting two terminal portions of an aorta (end-end anastomosis).

A critical aspect of anastomosis interventions is represented by the time necessary to suture the parts of the aorta to be connected.

Documents WO-A2-2010/026429, US-A1-2004/54405, CA-A1-2 366 703, US-A1-2011/118833, CN-A-106 725 682 and CN-A-106 580 402 describe connecting connectors for aorta according to the preamble of Claim 1.

Object of the present invention is solving the aforementioned prior art problems by providing a connector for aorta connection that allows two portions of an aorta to be connected reliably and quickly, in a short time to reduce suture line bleeding and reduce the time of circulation arrest, especially preserving the carotid functions.

The above and other objects and advantages of the invention, as will appear from the following description, are achieved with connectors for aorta connection such as those claimed in the independent claims.

Preferred embodiments and non-trivial variations of the present invention form the subject matter of the dependent claims.

It is understood that all attached claims form an integral part of this description.

It will be immediately obvious that countless variations and modifications can be made to what is described (for example relating to shape, dimensions, arrangements and parts with equivalent functionality) without departing from the scope of the invention as appears from the attached claims.

The present invention will be better described by preferred embodiments thereof, provided by way of example and not by way of limitation, with reference to the attached drawings, in which:
Fig. 1 shows a side sectional view of an example of a connector for aorta connection;
Fig. 2 shows a perspective view of a component of the connector for aorta connection of Fig. 1;
Fig. 3 shows a perspective view of the example of the connector for aorta connection of Fig. 1;
Fig. 4A, 4B, 4C, 4D show views of an example of a sealing band for the connector for aorta connection of Fig. 1;
Fig. 5 shows a perspective view of the example of a sealing band for the connector for aorta connection of Fig. 1;
Fig. 6 shows a side sectional view of an embodiment of the connector for aorta connection according to the present invention;
Fig. 7 shows a perspective view of the connector for aorta connection of Fig. 6;
Fig. 8 shows a side view of the connector for aorta connection of Fig. 6;
Fig. 9 is a detailed view of part of the connector of Fig. 6;
Fig. 10 is a perspective view of a component of the connector for aorta connection of Fig. 6; and
Fig. 11 is a partial sectional view of the component of Fig. 10.

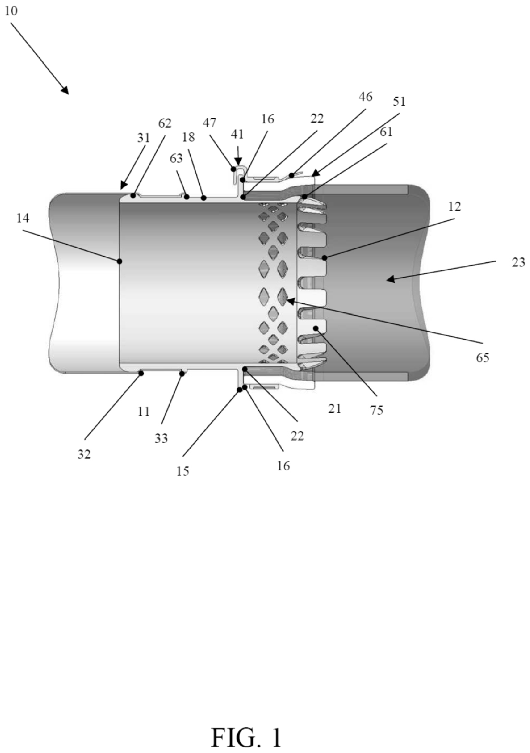

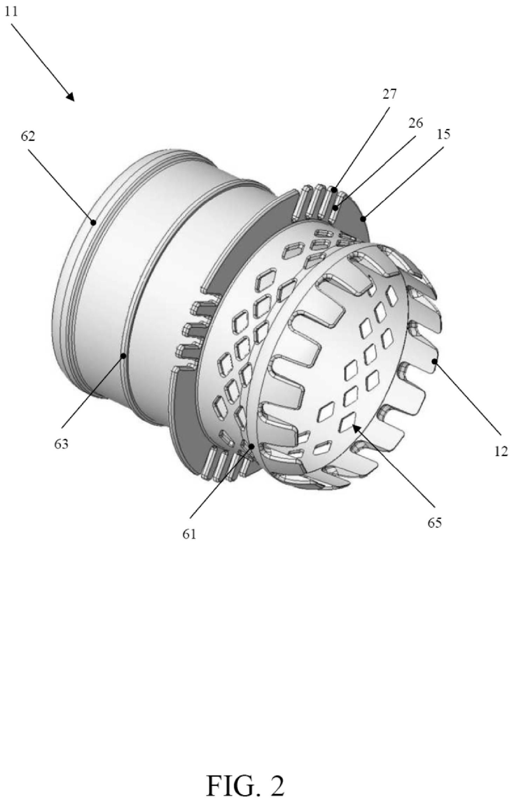

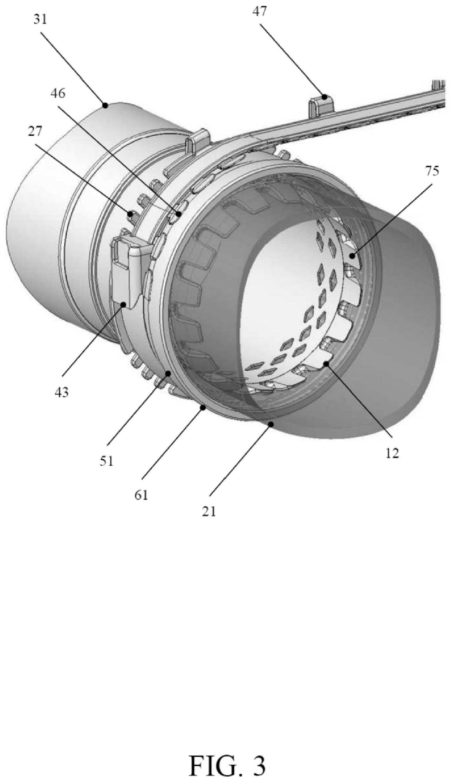

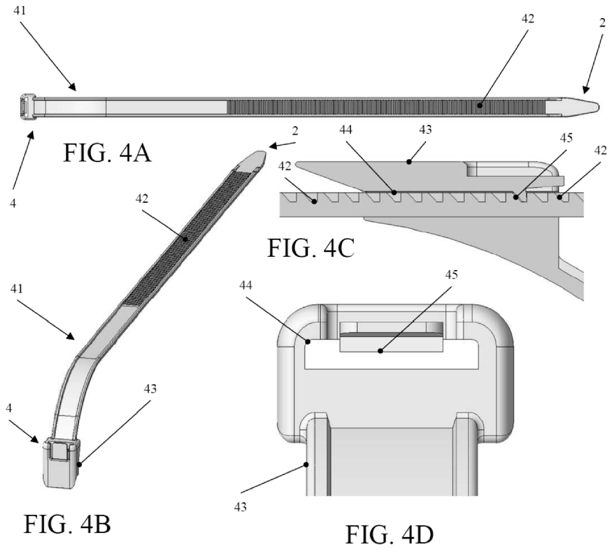

Referring to Figures 1 to 5, the connector for aorta connection 10 comprises:
- a duct 31 (prosthetic tube), for example made of plastic material, preferably of polyethylene terephthalate commercially known as Dacron;
- a sleeve 11 of tubular shape having a first inlet 12 configured to be connected to an aorta 21, and a second inlet 14 configured to be connected to the duct 31 (Dacron), said sleeve 11 further comprising: a first protruding annular portion 15 comprising an abutment surface 16 of an edge 22 of an opening 23 of the aorta 21 which must be connected, preferably said abutment surface 16 being substantially perpendicular to the axis and to an external surface 18 of the sleeve 11;
- fixing means 41 of the sleeve 11 to the aorta 21 configured to fix, by tightening their ends, the aorta 21 and the sleeve 11 in correspondence with the respective inlets 23, 12; preferably, the means 41 for fixing the sleeve 11 to the aorta 21 are a flexible elongated sealing band 41, for example made of polymeric material, comprising two ends 2, 4 and a plurality of toothed seats 42, made on one face of the sealing band 41 in correspondence with one end 2, aligned on at least one row, preferably on two parallel rows, said sealing band 41 comprising a fixing tab 43, made in correspondence with the other end 4, inside which the part of the sealing band 41 equipped with toothed seats 42 slides to tighten the sealing band 41 and with it the ends of the aorta 21 and the sleeve 11; the fixing tab 43 also includes a housing 44, inside which the part of the sealing band 41 equipped with the toothed seats 42 slides, and a fixing tooth 45 for each row of toothed seats 42 to block the sealing band 41 and fix the ends of the aorta 21 and of the sleeve 11 with the insertion of the fixing tooth 45 into the toothed seat 42.

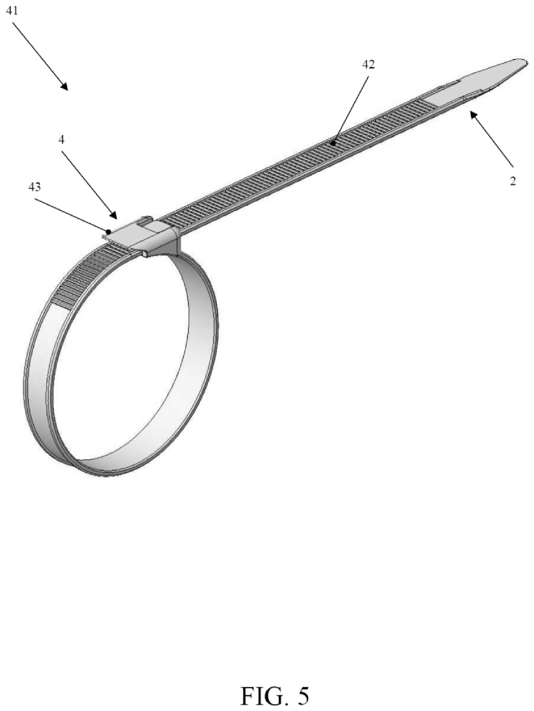

The end 4 of the sealing band 41 is curved in correspondence with the fixing tab 43, and the housing 44, inside which the part of the sealing band 41 equipped with the toothed seats 42 is inserted, develops in a direction tangential to the curve formed at the end 4 of the sealing band 41, so as to obtain a circular shape of the closed band (Fig. 5) to exert uniform pressure on the sleeve 11 and on the aorta 21.

The connector for aorta connection 10 comprises a ring 51, for example made of PTFE (polytetrafluoroethylene) coated fabric, mounted against the abutment surface 16 of the sleeve 11 interposed between the fixing means 41 and the aorta 21, in particular between the sealing band 41 and the aorta 21; preferably, the sealing band 41 includes a plurality of retaining tabs 46 made along a first side of the sealing band 41 to retain the ring 51 against the aorta 21. Preferably, the first protruding annular portion 15 has the shape of an external circular crown and includes recesses 26 made so as to obtain a protruding element 27, preferably four equidistant protruding elements 27, which can be used to bring the abutment surface 16 of the sleeve 11 into contact of the ring 51 and of the aorta 21 by means of four traction threads wrapped around said protruding elements 27 to keep the edge 22 of the mouth 23 of the aorta 21 against the abutment surface 16 of the first protruding annular portion 15.

The sleeve 11 includes a second annular portion 61 protruding in correspondence with the first inlet 12 so as to form an area on the external surface of the sleeve 11, included between said second protruding annular portion 61 and the abutment surface 16, in which to arrange the fixing means, in particular the sealing band 41, when the sleeve 11 is inserted into the aorta 21.

Preferably, the sleeve 11 includes a third protruding annular portion 62 in correspondence with the second inlet 14 to fix the sleeve 11 inside the inlet of the duct 31, blocking the third protruding annular portion 62 in a corresponding recess 32 obtained in the duct 31; preferably, the sleeve 11 includes a fourth protruding annular portion 63 spaced from the third annular portion 62 to fix the sleeve 11 inside the mouth of the duct 31, blocking the fourth protruding annular portion 63 in a corresponding recess 33 obtained in the duct 31; for example, the third 62 and the fourth 63 protruding annular portions are obtained by increasing the thickness of the wall of the sleeve 11.

The sealing band 41 comprises a plurality of hooking tabs 47 made along a second side of the sealing band 41 to hook the sealing band 41 onto the external circular crown 15, keeping it in the working position.

Preferably, the sleeve 11 includes aorta blocking openings 65 made in the area between the second protruding annular portion 61 and the abutment surface 16 to allow the aorta 21 to be supplied with blood; preferably, the sleeve 11 includes fins 75 made between the second protruding annular portion 61 and the first inlet 12, designed to improve the flow.

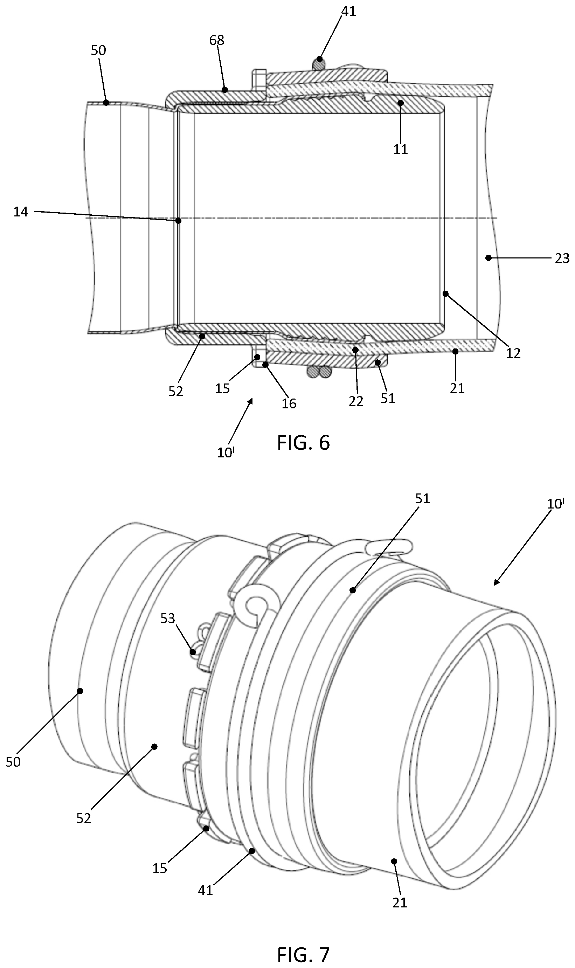

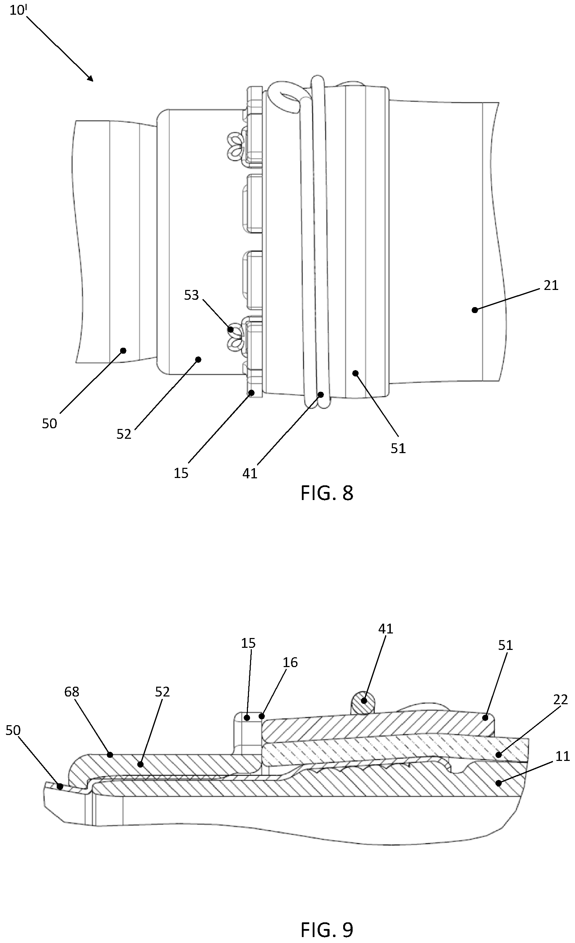

Referring to Figures 6 to 11, the connector for aorta connection 10' of the present invention comprises:
- a sleeve 11 of a tubular shape, preferably made of titanium, having a first inlet 12 configured to be connected to an aorta 21, and a second inlet 14 configured to be connected to a conduit (not shown), for example of the prosthetic tube type made of plastic material, preferably in polyethylene terephthalate commercially known as Dacron;
- a cylindrical covering element 50 made of plastic material, preferably in Dacron, said cylindrical covering element 50 being placed around and outside of said sleeve 11 on the side of said second inlet 14;
- an annular locking flange 52 placed around and outside of said cylindrical covering element 50 to lock it onto said sleeve 11, said annular locking flange 52 being preferably made of polyether ether ketone (also commercially known as Peek) and further comprising: a first protruding annular portion 15 comprising an abutment surface 16 of an edge 22 of a mouth 23 of the aorta 21 which must be connected, preferably said abutment surface 16 being made substantially perpendicular to the axis and to an external surface 68 of the annular locking flange 52;
- a stop ring 51, preferably made of Teflon, placed around and outside of said aorta 21 to fix said aorta 21 to said sleeve 11, said stop ring 51 being designed to abut against said abutment surface 16 together with and above said edge 22 of said aorta 21; and
- fixing means 41 of the retaining ring 51 to the aorta 21 configured to fix, by tightening their ends, the aorta 21 and the sleeve 11 in correspondence with the respective inlets 23, 12.

Preferably, the fixing means 41 are made with a spiral band, of the double elastic ring type, made of plastic or metallic material, preferably a super-elastic alloy of nickel and titanium commercially known as Nitinol.

Double suture threads 53 are also provided, fixed in a cross on the stop ring 51, which help the insertion of the aorta 21 onto the sleeve 11, as will be explained better below.

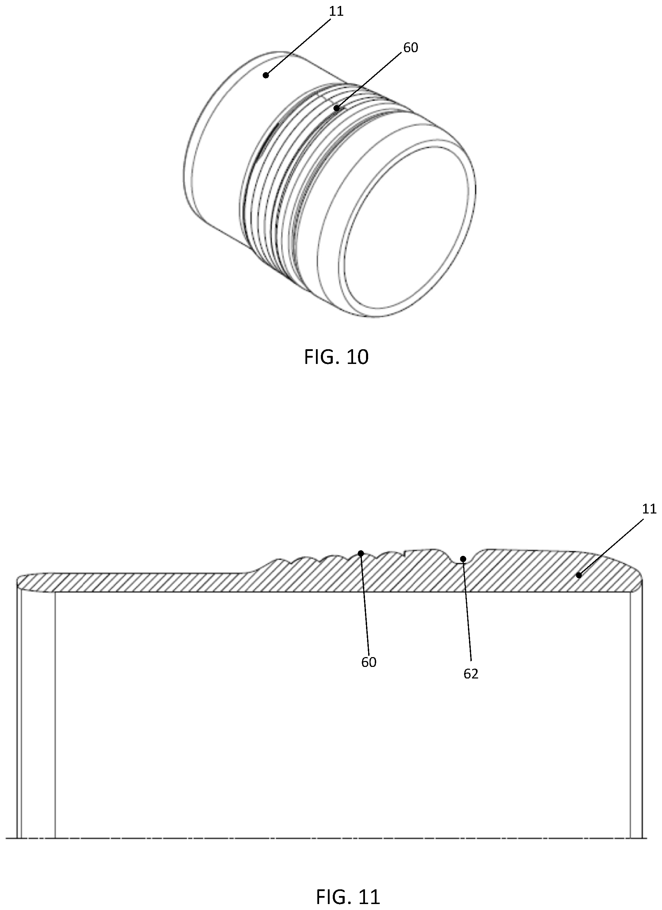

As better shown in Fig. 10 and 11, the sleeve 11 also includes a plurality of threads 60 for its screwing, and an external cavity 62 inside which the end of the cylindrical covering element 50 opposite the end of the second inlet 14 slides.

As regards the operation of the connector 10' of the invention, the sleeve 11 is initially covered with the covering element 50, which is screwed onto the sleeve 11 and blocked by the annular locking flange 52.

The aorta 21 is inserted on the bare part of the sleeve 11 to continue on the covering element 50 and come into contact with the annular locking flange 52, as can be better seen in Figure 6.

The insertion of the aorta 21 onto the sleeve 11 is facilitated by four double suture threads 53 fixed in a cross on the retaining ring 51 which embraces the aorta 21 itself.

The aorta 21 covered by the retaining ring 51 will come into contact with the annular locking flange 52 and everything is fixed by the four double threads 53 knotted on the annular locking flange 52.

The double elastic ring 41 blocks the aorta 21 covered by the annular locking flange 52 on the sleeve 11.

Advantageously, the aorta connecting connector allows two portions of a human body fluid conduit to be connected reliably and quickly, in a short time, reducing suture line bleeding and circulation arrest time.

## Claims

1. Connector (10') for aorta connection comprising:
- a sleeve (11) of a tubular shape, having a first mouth (12) configured to be connected to an aorta (21), and a second mouth (14) configured to be connected to a duct, for example of the prosthetic tube type;
- a cylindrical lining element (50), said cylindrical lining element (50) being placed around and outside of said sleeve (11) on the side of said second mouth (14);
- an annular locking flange (52) placed around and outside said cylindrical covering element (50) to lock it onto said sleeve (11), said annular locking flange (52) further comprising: a first protruding annular portion (15) comprising an abutment surface (16) of an edge (22) of an opening (23) of the aorta (21) which is to be connected;
- a retaining ring (51), placed around and outside of said aorta (21) to fix said aorta (21) to said sleeve (11), said retaining ring (51) being designed to abut against said abutment surface (16) together with and above said edge (22) of said aorta (21); and
- fixing means (41) of the retaining ring (51) to the aorta (21) configured to fix, by tightening the ends, the aorta (21) and the sleeve (11) in correspondence with the respective openings (23, 12).

2. Connector (10') for aorta connection according to claim 1, **characterized in that** said fixing means (41) are made with a spiral band, of the double elastic ring type, made of plastic or metallic material, preferably a super-elastic alloy of nickel and titanium or Nitinol.

3. Connector (10') for aorta connection according to claim 1 or 2, **characterized in that** it also comprises double suture threads (53) fixed in a cross on said retaining ring (51), which help the insertion of the aorta (21) on said sleeve (11).

4. Connector (10') for aorta connection according to any one of claims 1 to 3, **characterized in that** said sleeve (11) is made of titanium.

5. Connector (10') for aorta connection according to any one of claims 1 to 4, **characterized in that** said cylindrical covering element (50) is made of plastic material, preferably polyethylene terephthalate or Dacron.

6. Connector (10') for aorta connection according to any one of claims 1 to 5, **characterized in that** said abutment surface (16) is made substantially perpendicular to the axis and to an external surface (68) of the annular flange locking device (52).

7. Connector (10') for aorta connection according to any one of claims 1 to 6, **characterized in that** said annular locking flange (52) is made of polyether ether ketone or Peek.

8. Connector (10') for aorta connection according to any one of claims 1 to 7, **characterized in that** said retaining ring (51) is made of Teflon.

9. Connector (10') for aorta connection according to any one of claims 1 to 8, **characterized in that** said sleeve (11) further comprises a plurality of threads (60) for its screwing, and an external cavity (62) inside which the end of said cylindrical covering element (50) rests opposite the end of said second mouth (14).
